# EUROPEAN PATENT APPLICATION

(11) **EP 2 602 616 A1**
(43) Date of publication of application: **12.06.2013**
(21) Application number: 11814244.7
(22) Date of filing: 12.07.2011
(51) Int. Cl.: G01N 31/00, G01N 21/78, G01N 31/22

(54) **NITRIC OXIDE DETECTOR ELEMENT**

(30) Priority: 03.08.2010 JP 2010174334
(71) Applicant: Panasonic Healthcare Co., Ltd., Ehime 791-0395 (JP); National University Corporation Ehime University, Ehime 790-8577 (JP)
(72) Inventor: HIRANAKA, Kouichi, Osaka 540-6207 (JP); SADAOKA, Yoshihiko, Ehime 790-8577 (JP); ITAGAKI, Yoshiteru, Ehime 790-8577 (JP)
(74) Representative: TBK
(86) International application number: PCT/JP2011/003985
(87) International publication number: WO 2012/017604

(57) **Abstract**

Provided is a nitric oxide detection element which is capable of measuring a trace amount of NO gas contained in a gas in a scale of several ppb and of which the time degradation in performance is suppressed. The nitric oxide detection element includes at a surface thereof: a dye having a porphyrin skeleton and containing divalent cobalt as a central metal; and a radical scavenger. The nitric oxide detection element includes a substrate 12 and a sensing film 11 formed on a surface of the substrate 12. The sensing film 11 may contain the dye and the radical scavenger.

## Description

### Technical Field

The present invention relates to a nitric oxide detection element for use in detecting a trace amount of nitric oxide contained in a mixed gas.

### Background Art

Since the finding that nitric oxide (hereinafter, nitric oxide may also be referred to as NO) acts as an essential component of a muscle-relaxing factor, the physiological function of NO has been elucidated, and utilization of NO as a neurotransmitter or an infection marker has been under consideration.

In particular, analysis of NO gas in exhaled air has been attracting attention as a marker for airway inflammation caused by, for example, asthma or an allergy, the patients of which are increasing in recent years. This type of analysis allows noninvasive diagnosis of disease without imposing a burden on patients. The concentration of NO gas in exhaled air of normal adults is 2 ppb to 20 ppb, but is known to increase by a factor of approximately three in cases of airway inflammation caused by, for example, asthma or an allergy. The concentration of NO gas in exhaled air of children is lower than that of normal adults. Therefore, in cases of children, it is necessary to measure the concentration of a trace amount of NO gas in their exhaled air. If a simple and compact measurement device capable of measuring a trace concentration of NO gas is realized, the device can be used in determining the degree of airway inflammation of a patient or in determining treatment plans for asthma such as a dosage of asthma medication.

Conventionally, measurement of NO gas in exhaled air is performed in the following manner: cause a reaction between a patient's exhaled air and ozone under a reduced pressure, thereby causing excitation of part of NO gas contained in the exhaled air; and detect light that is emitted when the excited state returns to the ground state. However, such a chemiluminescence method requires expensive peripheral devices such as an ozone generator, and the maintenance of such devices is laborious.

An inexpensive and compact NO gas measurement device that is excellent in terms of gas selectivity, capable of quick measurement, and has high sensitivity is necessary for allowing asthma patients to measure the concentration of NO gas in their exhaled air everyday at a hospital or at home for self asthma management.

In recent years, there has been disclosed a method in which cobalt tetrakis(5-sulfothienyl)porphine (hereinafter, referred to as Co{T(5-ST)P}) contained in a silica film fabricated through a sol-gel process is reacted with NO gas in a vacuum chamber, and NO coordinated to Co{T(5-ST)P} is detected by using an ultraviolet and visible spectrophotometer (see, for example, Non Patent Literature 1).

In this method, in order to achieve necessary NO gas selectivity, an amorphous silica film containing Co{T(S-ST)P} is formed in the following manner: slowly hydrolyze ethyl silicate for 24 hours in the presence of Co{T(S-ST)P}; apply a resultant solution onto a glass substrate; and dry the glass substrate. The film formed in this manner is used as a NO sensor. This method has succeeded in detecting 17 ppm of NO gas with a sensor temperature of 200 °C.

Further, there has been disclosed a method in which a porous glass plate is immersed in a chloroform solution containing cobalt tetraphenylporphyrin (5,10,15,20-tetraphenyl-21H,23H-porphyrin cobalt (hereinafter, referred to as CoTPP)), and is then dried. In this manner, a NO sensor in which the porous glass plate has CoTPP supported thereon is formed (see, for example, Non Patent Literature 2). According to the disclosure, the sensor is placed in a reactor that has been vacuum-evacuated by an oil diffusion pump, and NO gas is detected by means of an infrared spectrophotometer or an ultraviolet and visible spectrophotometer.

Still further, there has been disclosed a method of optically detecting NO gas by using a NO sensor which is a sol-gel glass having cytochrome c included therein (see, for example, Patent Literature 1). It is disclosed that in a case where cytochrome c, which is a protein, is used for a NO sensor, time degradation of the protein can be prevented by conserving the sensor in a low oxygen concentration environment or in an oxygen-free environment (see Patent Literature 2).

### Citation List

### Patent Literature

PTL 1: Japanese National Phase PCT Laid-Open Publication No. 2008-530527
PTL 2: Japanese National Phase PCT Laid-Open Publication No. 2008-530534

### Non Patent Literature

NPL 1: Hiromichi ARAI et al., "Optical Detection of Nitrogen Monoxide by Metal Porphine Dispersed Amorphous Silica Film", CHEMISTRY LETTERS of the Chemical Society of Japan, 1988, pp. 521-524
NPL 2: Makoto MIYAMOTO and Yoshio HANAZATO, "Nitrogen Monoxide Adsorption and Contact Decomposition Properties of Co(II) Complexes", Journal of the Chemical Society of Japan, 1998, No5, pp. 338-345

### Summary of Invention

### Technical Problem

Conventional NO gas sensors using a porphyrin have a problem that when NO detection is performed by repeatedly irradiating a NO gas sensor with light, time degradation in the sensor's performance is likely to occur. The term "degradation" herein refers to a decrease in the sensor's NO sensitivity with respect to the magnitude of a change from which a trace amount of NO gas can be detected.

An object of the present invention is to provide a nitric oxide detection element which is capable of measuring a trace amount of NO gas contained in a gas in a scale of several ppb and of which the time degradation in performance is suppressed.

### Solution to Problem

In order to solve the conventional problems, the present invention relates to a nitric oxide detection element. The nitric oxide detection element includes at a surface thereof: a dye having a porphyrin skeleton and containing divalent cobalt as a central metal; and a radical scavenger.

In the present invention, preferably, the nitric oxide detection element includes a substrate and a sensing film formed on a surface of the substrate, and the dye and the radical scavenger are contained in the sensing film. Specifically, the sensing film may be formed from nitric oxide sensing particles and a polymer adhesive, and the nitric oxide sensing particles may be formed in such a manner that the dye and the radical scavenger are adsorbed to surfaces of inorganic particles. Alternatively, the sensing film may be formed in such a manner that the dye and the radical scavenger are dispersed within the polymer adhesive.

In the present invention, preferably, the nitric oxide detection element further includes a support, and the dye and the radical scavenger are supported on a surface of the support.

The present invention also relates to a nitric oxide detector. The nitric oxide detector includes: the nitric oxide detection element; a gas introducing part configured to cause a measurement gas, which possibly contains nitric oxide, to come into contact with the surface of the nitric oxide detection element; a phototransmitter configured to emit light to the surface; and a photoreceiver configured to receive light reflected by the surface or light transmitted through the surface.

The present invention further relates to a nitric oxide detection method. The nitric oxide detection method includes: a first step of initializing the above-described nitric oxide detection element; a second step of emitting light to the surface of the nitric oxide detection element and measuring an optical absorptance of the surface after the first step; a third step of causing a measurement gas, which possibly contains nitric oxide, to come into contact with the surface of the nitric oxide detection element after the second step; a fourth step of emitting light to the surface and measuring an optical absorptance of the surface after the third step; and a fifth step of comparing the optical absorptance obtained in the fourth step and the optical absorptance obtained in the second step to determine a nitric oxide concentration in the measurement gas.

### Advantageous Effects of Invention

The nitric oxide detection element according to the present invention suppresses time degradation in performance that occurs when NO detections involving repeated irradiation of light are performed, and is capable of meaningfully measuring the concentration of a trace amount of NO gas with high reliability.

### Brief Description of Drawings

[Fig. 1] Fig. 1 is a cross-sectional conceptual diagram showing a nitric oxide detection element according to Embodiment 1 of the present invention.
[Fig. 2] Fig. 2 is an enlarged conceptual diagram showing a relationship among materials forming a sensing film 11 of Fig. 1.
[Fig. 3] Fig. 3 is a conceptual diagram showing a nitric oxide detector which includes a nitric oxide detection element 10A according to the present invention.
[Fig. 4] Fig. 4 is a UV-Vis reflection spectrum shown by the nitric oxide detection element according to Embodiment 1.
[Fig. 5] Fig. 5 is a graph showing changes in optical reflectance between before and after the nitric oxide detection element according to Embodiment 1 is exposed to 1 ppm of NO gas.
[Fig. 6A and Fig. 6B] Fig. 6A and Fig. 6B are graphs showing a relationship between an optical absorptance and a NO sensitivity that are obtained from measurement performed when the nitric oxide detection element according to Embodiment 1 is exposed to 1 ppm of NO gas, and a relationship between the optical absorptance and a NO response time that are obtained from the measurement performed when the nitric oxide detection element according to Embodiment 1 is exposed to 1 ppm of NO gas.
[Fig. 7A and Fig. 7B] Fig. 7A and Fig. 7B are graphs showing a relationship between the number of cobalt atoms per unit area and the NO sensitivity that are obtained from the measurement performed when the nitric oxide detection element according to Embodiment 1 is exposed to 1 ppm of NO gas, and a relationship between the number of cobalt atoms per unit area and the NO response time that are obtained from the measurement performed when the nitric oxide detection element according to Embodiment 1 is exposed to 1 ppm of NO gas.
[Fig. 8A and Fig. 8B] Fig. 8A and Fig. 8B are graphs showing temporal changes (in a short period of time) in an active dye rate and a normalized differential optical reflectance of the nitric oxide detection element according to Embodiment 1.
[Fig. 9A and Fig. 9B] Fig. 9A and Fig. 9B are graphs showing temporal changes (in a long period of time) in the active dye rate and the normalized differential optical reflectance of the nitric oxide detection element according to Embodiment 1.
[Fig. 10] Fig. 10 is an enlarged conceptual diagram showing a relationship among materials forming a nitric oxide detection element according to Embodiment 2 of the present invention.
[Fig. 11] Fig. 11 is an enlarged conceptual diagram showing a relationship among materials forming a nitric oxide detection element according to Embodiment 3 of the present invention.

### Description of Embodiments

Hereinafter, a nitric oxide detection element and a fabrication method thereof according to the present invention are described based on embodiments.

### (Embodiment 1)

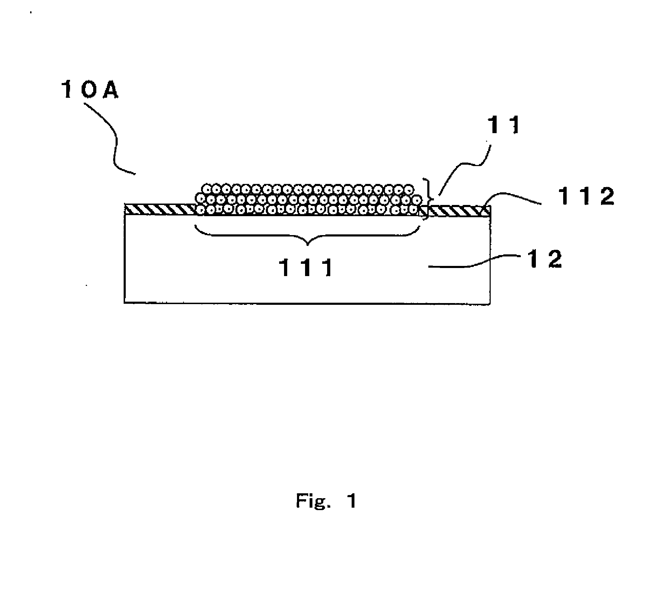

Fig. 1 is a cross-sectional conceptual diagram showing a nitric oxide detection element 10A according to Embodiment 1. As shown in Fig. 1, a sensing film 11 is fixed on the surface of a substrate 12. Patterning is performed in advance on the surface of the substrate 12, and the surface is divided into a sensing film portion 111 and a peripheral portion 112. The sensing film 11 is formed on the sensing film portion 111.

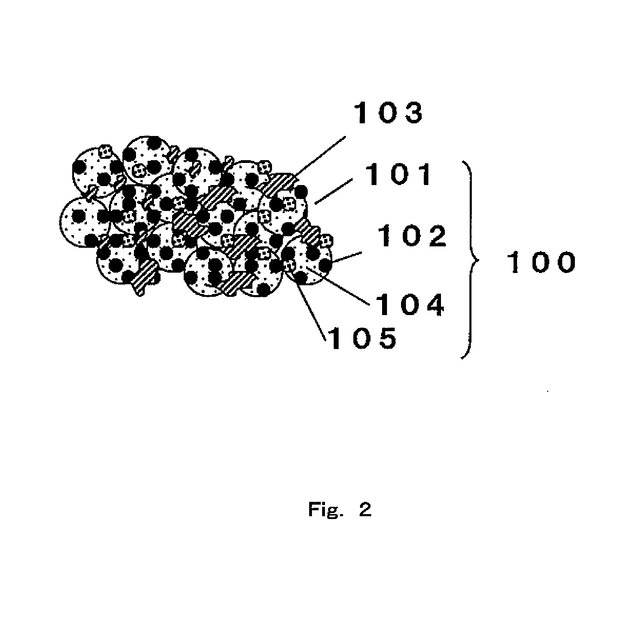

Fig. 2 is an enlarged conceptual diagram showing a relationship among materials forming the sensing film 11 of Fig. 1. As shown in Fig. 2, a dye 102 and a radical scavenger 105 are supported on the surfaces of inorganic particles 101, and thus nitric oxide sensing particles 100 are formed. Preferably, a non-ionic surfactant 104 is used, the presence of which suppresses the dye 102 and the radical scavenger 105 from aggregating together. Accordingly, the dye 102 and the radical scavenger 105 are supported on the surfaces of the inorganic particles 101 in a dispersed manner. The nitric oxide sensing particles 100 are bound to each other via a polymer adhesive 103 to form a single body as the sensing film 11. The sensing film 11 is adhered to, and thereby fixed to, the surface of the substrate 12 also via the polymer adhesive 103.

Hereinafter, component materials forming the nitric oxide detection element are described in detail.

### (Dye)

The present invention uses the dye 102 which contains a porphyrin complex. The porphyrin complex herein has a porphyrin skeleton which contains a metal at its center. The porphyrin skeleton may be modified by various substituents.

The light absorption spectrum of the porphyrin complex indicates Soret band (B-band) absorption in an optical wavelength region of 400 nm to 450 nm (ultraviolet light region) and Q-band absorption in an optical wavelength region of no less than 500 nm (visible light region). In selecting the porphyrin complex, a relationship between a molar absorption coefficient and a NO sensitivity may be taken into consideration. The oxidation-reduction potential of the central metal of the porphyrin complex affects binding between the central metal and NO gas. The Porphyrins, Volume III, edited by David Dolphin, Academic Press, Inc., pp. 14-15, teaches that generally speaking, the molar absorption coefficient in the Soret band is approximately 10⁵(M⁻¹·cm⁻¹) and the molar absorption coefficient in the Q-band is approximately 103(M⁻¹·cm⁻¹).

The inventors of the present invention consider that in order to detect NO gas that is contained in exhaled air in a scale of several ppb to a few hundred of ppb, it is preferred to utilize changes in the absorption spectrum in the Soret band which has a large molar absorption coefficient, and studied this matter from various aspects. The molar absorption coefficient proportionally increases in accordance with an increase in NO sensitivity, that is, an increase in NO gas concentration. For this reason, it is preferred to use, among porphyrin complexes, a porphyrin complex having a symmetric molecular structure. The higher the degree of symmetry of the molecular structure of the porphyrin complex, the higher the absorption in the Soret band of the porphyrin complex, whereas the lower the degree of symmetry of the molecular structure of the porphyrin complex, the lower the absorption in the Soret band of the porphyrin complex. Although the absorption in the Q-band is less affected by the molecular structure of the porphyrin complex than the absorption in the Soret band, the molar absorption coefficient in the Q-band is equal to or less than 10⁴(M⁻¹·cm⁻¹), which is low.

Conceivable ways of increasing the NO sensitivity of the porphyrin complex include the following: (1) suitably select the central metal of the porphyrin complex and (2) change the substituents of the porphyrin skeleton, thereby donating electrons to a macrocyclic π conjugated system at the center of the porphyrin structure (i.e., electron-donating) and withdrawing electrons from the π conjugated system (i.e., electron-withdrawing).

### (1) Central Metal

NO sensitivities of porphyrin complexes that contain iron (Fe), Mn (manganese), cobalt (Co), Ni (nickel), and Zn (zinc) as their respective central metals were examined. The results indicated that the porphyrin complexes containing respective central metal elements other than cobalt showed poor reactivity with NO gas, that is, they showed low NO sensitivity. In contrast, it was found that the porphyrin complex containing cobalt as a central metal showed high NO sensitivity. In the case of Co(II)TPP, the molar absorption coefficient in the Soret band is 2.8x10⁵(M⁻¹·cm⁻¹) and the molar absorption coefficient in the Q-band is 1.2x10⁴(M⁻¹·cm⁻¹). Accordingly, the present invention uses a porphyrin complex that contains divalent cobalt as a central metal. It is presumed that a difference in NO gas reactivity between the porphyrin complex containing divalent cobalt and the other porphyrin complexes containing various respective metals depends on a difference between the oxidation-reduction potential of a central metal and the oxidation-reduction potential of NO gas.

### (2) Substituent

Next, a porphyrin complex of which the central metal is cobalt and of which the molecular structure has a high degree of symmetry was selected, and an influence of the substituents of the porphyrin skeleton on the NO sensitivity was examined. The structure of a cobalt porphyrin complex, CoTP(Xi)P, used in the examination is represented by a chemical formula shown below.

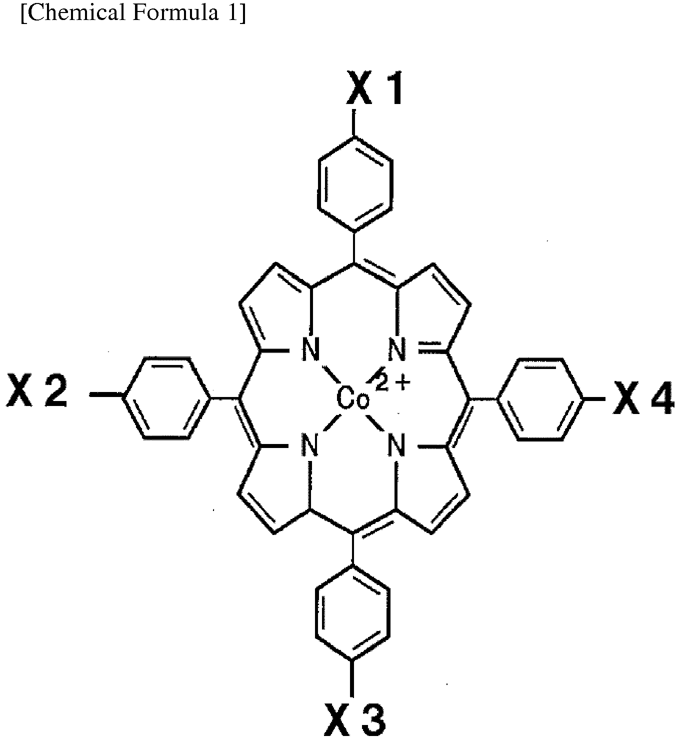

In the chemical formula, X1, X2, X3, and X4 represent hydrogen (-H), or a methoxy group (-OCH₃), or a hydroxyl group (-OH).

CoTP(Xi)P represented by the above formula is a porphyrin complex that contains divalent cobalt as a central metal and that has four phenyl groups on the outside of its porphyrin skeleton. Xi (i = an integer from 1 to 4) represents substituents bound to the phenyl groups, and the substituents are selected from hydrogen (-H), the methoxy group (-OCH₃), or the hydroxyl group (-OH).

In a case where Xi = -H, the cobalt porphyrin complex is cobalt tetraphenylporphyrin (5,10,15,20-tetraphenyl-21H,23H-porphyrin cobalt (CoTPP). In a case where Xi = -OCH₃, the cobalt porphyrin complex is cobalt tetramethoxyphenylporphyrin (5,10,15,20-terta(4-methoxyphenyl)-21H,23H-porphyrin cobalt (CoTP(4-OCH₃)P)). The same advantageous effects can be obtained by using a mixture of these compounds. In a case where Xi = -OH, the cobalt porphyrin complex is cobalt tetrahydroxyphenylporphyrin (5,10,15,20-tetra(4-hydroxyphenyl)-21H,23H-porphyrin cobalt (CoTP(4-OH)P)).

From the examination, it has been found that if the substituents of the porphyrin complex are hydrogen, the methoxy group, or the hydroxyl group, then the NO sensitivity indicated by the porphyrin complex increases in the order of hydrogen, the methoxy group, and the hydroxyl group.

### (Radical Scavenger)

The present invention uses the radical scavenger 105 together with the dye 102. A nitron compound or a nitroso compound may be used as the radical scavenger 105. Using a nitron compound as the radical scavenger 105 is particularly preferred.

In a case where the nitric oxide detection element according to the present invention is used to detect NO in exhaled air, the exhaled air containing NO gas which is a subject gas is sampled while irradiating the sensing film 11 with detection light which has an optical wavelength not shorter than 400 nm and not longer than 450 nm, and the sampled exhaled air is caused to react with the sensing film 11. The inventors of the present invention have found that at the time of the NO detection, a free radical such as superoxide anion radical or hydroxyl radical occurs when oxygen and/or steam contained in the exhaled air react with the detection light, and such a free radical oxidizes and degrades a cobalt porphyrin. The inventors of the present invention consider that this mechanism causes time degradation in the performance of the nitric oxide detection element.

In this respect, the inventors of the present invention have found that such oxidation of the cobalt porphyrin and time degradation in the performance of the nitric oxide detection element are suppressed if, in the nitric oxide detection element, a radical scavenger effective for scavenging free radicals is disposed closely to the dye in a dispersed manner, and thus arrived at the present invention.

In selecting a radical scavenger to be used in the present invention, its reactivity with the dye, its solubility with a solvent for dissolving the dye, and its influence on NO gas detection may be taken into consideration. In general, it is particularly preferred to use a nitron compound as a radical scavenger since its reactivity with oxy radicals is high. Among nitron compounds, phenyl N-tert-butylnitrone (abbreviated as PBN, available from Tokyo Chemical Industry Co., Ltd.) is particularly preferred. Alternatively, 5,5-dimethyl-1-pyrroline-N-oxide (abbreviated as DMPO) or 3,5-di-t-butyl-4-hydroxy-phenyl-N-t-butylnitrone (abbreviated as BHPBN) may be used.

### (Non-Ionic Surfactant)

The non-ionic surfactant 104 may be used for the purpose of suppressing the dye 102 and the radical scavenger 105 from aggregating together and sufficiently dispersing the dye 102 and the radical scavenger 105. For such purposes, the non-ionic surfactant is preferably one, the hydrophilic-lipophilic balance (HLB value: a numerical value indicating the degree of affinity of a surfactant for water and oil) of which is not lower than 13 and not higher than 15. As a nonlimiting example, use of Triton X-100 (registered trademark, available from GE Healthcare UK Ltd.), the HLB value of which is 13.5, is preferred.

As an alternative, a hydrophilic non-ionic surfactant and a lipophilic non-ionic surfactant may be mixed together at a suitable composition ratio to form a non-ionic surfactant mixture that indicates an HLB value suitable for use in the present invention. The non-ionic surfactant mixture thus prepared may be used. In this case, for example, TWEEN80 (available from Tokyo Chemical Industry Co., Ltd.) may be selected as a hydrophilic non-ionic surfactant, and SPAN80 (available from Tokyo Chemical Industry Co., Ltd.) may be selected as a lipophilic non-ionic surfactant. By mixing these surfactants together at a suitably adjusted composition ratio, the same surfactant performance as that of Triton X-100 can be achieved.

### (Inorganic Particles)

Although the inorganic particles 101 are not limited to particular inorganic particles, inorganic particles such as silica particles or α-alumina particles are preferred. A mixture of silica particles and α-alumina particles may also be used.

Preferably, the inorganic particles used herein are water-repellent treated. A method used for treating the inorganic particles to have water repellency may be a publicly known conventional method. For example, water-repellent treated inorganic particles 101 can be obtained by causing a chemical reaction between a silane coupling agent and the aforementioned particles, or by boiling the particles with silicone oil. In a case where such water-repellent treated inorganic particles are used in the present embodiment, when the inorganic particles are mixed with a solvent in a fabrication process of the nitric oxide detection element, the inorganic particles are well dispersed in the solvent and do not easily settle out. Accordingly, the dye 102 is sufficiently dispersed and thereby suppressed from aggregating, which makes it possible to improve the NO sensitivity of the nitric oxide detection element to be fabricated.

Preferably, the inorganic particles 101 have a particle diameter of 6 µm to 12 µm. The particle diameter can be measured by using a publicly known particle size distribution measurement apparatus, for example, LA-950 (available from HORIBA, Ltd.). In the measurement, an upper limit cumulative frequency median diameter d90 and a lower limit cumulative frequency median diameter d10 in a cumulative frequency distribution curve of particle size distribution obtained through a method compliant with JIS K 5600-9-3 (2006) are deemed to be an upper limit particle diameter and a lower limit particle diameter. Specifically, the particle diameter is not less than a cumulative frequency median diameter d10 of 6 µm and not greater than a cumulative frequency median diameter d90 of 12 µm in particle size measurement using a publicly known particle size distribution measurement apparatus (median diameters are such that d₁₀ = 6 µm and d₉₀ = 12 µm, and a mode diameter is 9 µm; hereinafter, the particle diameter is in the range of 6 µm to 12 µm, and an average particle diameter is the aforementioned mode diameter). In the present invention, silica particles, α-alumina particles, or a mixture of silica and α-alumina particles of which the particle diameter is in the aforementioned particle diameter range may be suitably used.

When particles having a diameter less than 6 µm are adhered to each other, they tend to detach from each other since the adhesion between the particles is weak. Moreover, in the case of using such particles having a diameter less than 6 µm, when the substrate 12 and the nitric oxide sensing particles 100 are adhered to each other, the adhesion tends to be weak. In this case, in order to obtain sufficient adhesion, it is desired that the polymer adhesive 103 is disposed between the particles, as well as between the substrate and the particles, and also, it is desired to increase the amount of usage of the polymer adhesive. However, an increase in the amount of usage of the polymer adhesive causes an extended NO response time, resulting in less prompt NO response. Therefore, it is preferred that the particle diameter is 6 µm or greater. If the particle diameter exceeds 12 µm, the NO sensitivity slightly increases. In this case, however, the NO response time is extended. The reason for this is that the greater the particle diameter, the more the particles tend to aggregate. Therefore, in order to satisfy both the NO sensitivity and the NO response time, it is preferred that the diameter of the water-repellent treated particles is not less than 6 µm and not greater than 12 µm.

### (Polymer Adhesive)

The polymer adhesive 103 acts as an adhesive for adhering the nitric oxide sensing particles to each other to form the sensing film, and as an adhesive for adhering the nitric oxide sensing particles to the substrate 12.

Preferably, the glass transition temperature (hereinafter, referred to as Tg) of the polymer adhesive is in a range from -150 °C to 150 °C. Polymer adhesives having the glass transition temperature within this range are excellent in terms of gas permeability. The NO response time can be reduced when a polymer adhesive having excellent gas permeability is used. Adhesion of the sensing film 11 to the substrate 12 is insufficient when Tg is lower than -150 °C. Gas permeability of the polymer adhesive becomes insufficient and the NO response time becomes extended when Tg exceeds 150 °C.

Moreover, in fabricating the sensing film of the nitric oxide detection element, the polymer adhesive is preferably transparent against the detection light. In the case of using, as the detection light, light having an optical wavelength not shorter than 400 nm and not longer than 450 nm, the polymer adhesive is preferably transparent in an optical wavelength region of 400 nm to 450 nm.

Preferred examples of such a polymer adhesive include: hydroxypropylcellulose (referred to as HPC, Tg = 19 °C to 125 °C, and Tg depends on a molecular weight); polycarbonate-based urethane resin (available from Meisei Chemical Works, Ltd., Tg = -30 °C to 130 °C); polyethylene glycol (referred to as PEG, Tg = -115 °C to 86 °C); polyethylene oxide (referred to as PEO, Tg = -53 °C); acrylic resins such as polymethylisobutyl methacrylate (Tg = 48 °C), poly(methyl acrylate) (Tg = 66 °C), and polyacrylonitrile (Tg = 97 °C); vinyl resins such as polystyrene (Tg = 100 °C), polyvinyl chloride (Tg = 81 °C), and polyvinyl alcohol (Tg = 85 °C); polydimethylsiloxane (Tg = -123 °C); ethyl cellulose (Tg = 43 °C); and biodegradable plastics such as polycaprolactone (Tg = -62 °C), polybutylene succinate (Tg = -33 °C), and polybutylene succinate adipate (Tg = -42 °C). It should be noted that examples of the polymer adhesive also include copolymers of those in the above examples that can be copolymerized. Examples of the polymer adhesive further include modified products of those in the above examples that can be modified by using a side-chain substitution product for the purpose of improving a refractive index, thermal resistance, or the like.

Any of the above polymer adhesives may be used in combination with a plasticizer, aiming at improving flowability. For example, dioctyl phthalate, which is a plasticizer, may be mixed into ethyl cellulose (Tg = 43 °C), and the resultant mixture may be used as a substitute for PEO.

### (Substrate)

Preferably, the substrate 12 is sheet-shaped and is formed of a thermal-resistant material that reflects the detection light or allows the detection light to pass through. If light having an optical wavelength not shorter than 400 nm and not longer than 450 nm is used as the detection light, it is preferred that the substrate 12 is formed of a material that reflects the light having an optical wavelength in the range of 400 nm to 450 nm or allows the light to pass through.

Examples of such a substrate include: plastic substrates such as thermal-resistant films, including a polyethylene terephthalate film (PET), a polyethylene naphthalate film (PEN, registered trademark, available from DuPont Teijin Films), and an ARTON film (ARTON, registered trademark, JSR Corporation); ceramic substrates such as a glass substrate, a quartz substrate, and an alumina substrate; metal substrates containing aluminum or silver as a main component; paper; woven fabrics; and nonwoven fabrics. A composite of these materials may also be used as the substrate. A metal film containing silver or aluminum as a main component may be formed on the surface of the substrate 12. Forming the sensing film 11 on the metal film makes it possible to reduce power consumption of a light source 16 of a phototransmitter.

Hereinafter, usage manner of the nitric oxide detection element formed as described above is described.

The weight of the nitric oxide sensing particles 100 per unit area of the substrate 12 is preferably 0.2 mg/cm² to 2.0 mg/cm². If the weight per unit area is less than 0.2 mg/cm², then a change in light spectrum with respect to a trace amount of NO gas decreases, resulting in insufficient NO sensitivity. If the weight per unit area exceeds 2.0 mg/cm², then adhesion of the sensing film 11 becomes weak, resulting in an increased possibility of occurrence of cracks in the sensing film 11.

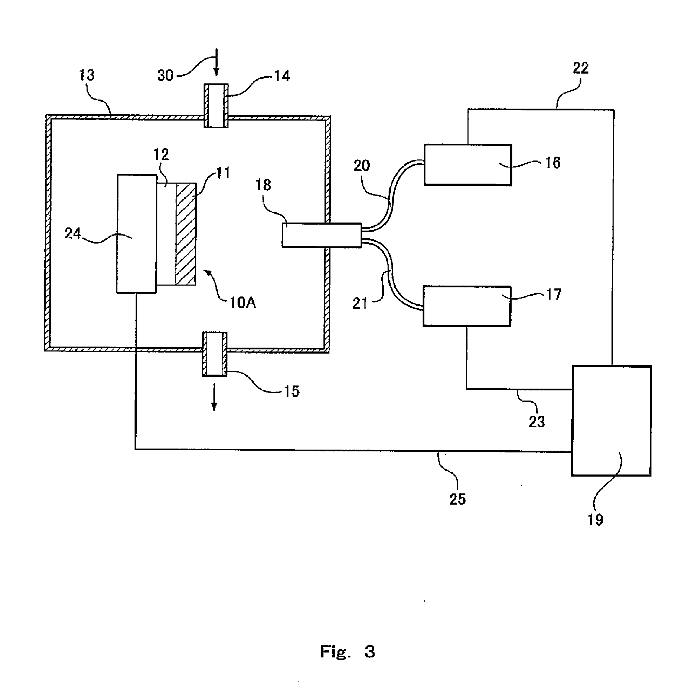

Fig. 3 is a conceptual diagram showing a configuration of a nitric oxide detector which includes the nitric oxide detection element 10A according to the present invention.

The nitric oxide detection element 10A, which includes the sensing film 11 and the substrate 12, is disposed within a measurement cell 13. Measurement gas 30, which possibly contains nitric oxide (NO), is introduced into the measurement cell 13 through a gas introducing inlet 14 and is then discharged from a gas exhaust outlet 15. During this process, the surface of the sensing film 11 is exposed to the measurement gas 30.

The nitric oxide measurement device shown in Fig. 3 is of a light-reflection type. For the purpose of detecting a change in optical properties of the sensing film 11 before and after the sensing film 11 is exposed to the measurement gas 30, a photo transmitter/photoreceiver 18 is disposed such that the photo transmitter/photoreceiver 18 is opposed to the nitric oxide detection element 10A in the light-reflection type nitric oxide measurement device. The photo transmitter/photoreceiver 18 is connected to the light source 16 via an optical fiber 20, and is connected to a photodetector 17 via another optical fiber 21. Light from the light source 16 (preferably, light having an optical wavelength range including 400 nm to 450 nm) is emitted from the photo transmitter/photoreceiver 18 and perpendicularly falls on the sensing film 11 of the nitric oxide detection element 10A. The light is reflected by the surface of the sensing film 11 and is then incident on the photo transmitter/photoreceiver 18. Thereafter, the light is guided to the photodetector 17.

As one example, the photodetector 17 includes a CCD and a diffraction grating such as a prism or a grating. Alternatively, the photodetector 17 may include an optical band-pass filter, a silicon photodiode, a photocurrent-voltage conversion circuit, and an amplifier circuit (not shown). Regardless of which of the above configurations is applied to the photodetector 17, the detection light is converted into a light detection signal corresponding to the amount of reflected light, and then measured.

Since a temperature controller 24 is provided within the measurement cell 13, the internal temperature of the measurement cell 13 can be controlled. The temperature controller 24 includes a heater and a thermocouple for use in temperature detection (not shown). The light source 16, the photodetector 17, and the temperature controller 24 are connected to the measurement controller 19 via control lines 22, 23, and 25, respectively, so that the operations of the light source 16, the photodetector 17, and the temperature controller 24 can be controlled.

Although in the above description the phototransmitter and the photoreceiver are integrally formed together, the phototransmitter and the photoreceiver may be provided as separate components. In a case where the substrate is formed of a transparent material, the phototransmitter and the photoreceiver may be disposed such that they face each other with the nitric oxide detection element 10A positioned therebetween.

Next, a description is given of an example of a fabrication method of the nitric oxide detection element 10A.

### (1) Preparation of Polymer Adhesive Solution

A polymer adhesive solution is prepared by using "HPC" (available from Sigma-Aldrich Co.) as the polymer adhesive 103 and using an alcohol-based solvent (e.g., methyl alcohol, ethyl alcohol, isopropyl alcohol, or a mixed solvent of these) as a first solvent. The polymer adhesive used here is not limited to HPC, but may be any of the aforementioned compounds. Preferably, the concentration of the polymer adhesive solution is adjusted such that the weight ratio of the polymer adhesive to the inorganic particles is 0.07 g/g to 0.20 g/g. Specifically, HPC is dissolved into the first solvent such that the HPC concentration is 6 mg/mL. In addition, a droplet amount of the polymer adhesive solution and a droplet amount of a dye-containing preparation solution are adjusted. The dye-containing preparation solution is described below.

### (2) Preparation of Dye-Containing Preparation Solution

As one example, a dye CoTP(4-OCH₃)P [cobalt tetramethoxyphenylporphyrin], water-repellent treated silica particles having a diameter of 6 µm to 12 µm (NIPGEL (registered trademark) available from Tosoh Silica Corporation), a non-ionic surfactant Triton X-100 having an HLB value of 13.5, and a radical scavenger PBN are mixed into a halogen-based solvent (e.g., chloroform, dichloromethane, etc.) which is a second solvent, and thus a preparation solution is prepared. For example, the amounts of respective components contained in the preparation solution are as follows: in the preparation solution, the molarity of CoTP(4-OCH₃)₄P is 3.3×10⁻⁵ mol/L to 3.3×10⁻⁴ mol/L, the concentration of the water-repellent treated silica particles is 10 mg/mL to 100 mg/mL, and the concentration of the non-ionic surfactant is 0.16 mg/mL to 30 mg/mL. The molar weight ratio between the dye and the water-repellent treated silica particles is 1.0×10⁻⁶ mol/g to 1.0×10⁻⁵ mol/g, and the molar weight ratio between the dye and the non-ionic surfactant is 3.0×10⁻⁶ mol/g to 3.0×10⁻⁴ mol/g. The weight ratio of the non-ionic surfactant to the inorganic particles is 0.05 g/g to 1g/g. The molar ratio of the dye to the radical scavenger is 0.3 to 100.

### (3) Patterning on Substrate

Preferably, the sensing film portion 111 is formed in advance by patterning on the surface of the substrate 12. By performing the patterning, a variation in the area of the sensing film can be reduced, which allows precise measurement of a trace amount of nitric oxide gas. Although photolithography or a printing process used in semiconductor processing may be used for the patterning, the patterning method is not limited thereto.

In the patterning, it is preferred that the peripheral portion 112 surrounding the sensing film portion 111 is liquid-repellent treated, and the sensing film portion 111 is lyophilic-treated. Varying the substrate surface properties between the sensing film portion 111 and the peripheral portion 112 in this manner allows the sensing film 11 to be formed with high precision, which makes it possible to reduce a variation in the NO sensitivity of the sensing film 11.

Specific examples of a method of the patterning include methods (i) and (ii) as described below. In the method (i), the peripheral portion 112 is coated with a photoresist or a metal, such that only the sensing film portion 111 is exposed, and then plasma etching is performed on the sensing film portion 111 by using a mixed gas containing oxygen gas as a main component, such that irregularity is formed on the surface of the sensing film portion 111. In the method (ii), a fluorine resin coating such as FS-1010 (registered trademark, Fluoro Technology), or a silicone oil coating, is formed on the peripheral portion 112. A particularly preferred method is that a PEN film substrate is used as the substrate 12 and an FS-1010 fluorine resin coating is formed on the peripheral portion 112. With any of the above methods, the sensing film portion 111 can be readily made lyophilic as compared to the peripheral portion 112. Such surface properties of the substrate 12 can be confirmed, for example, by a method in which pure water is dripped onto the surface and the pure water contact angle is measured by using a FACE contact angle meter of CA-C series available from Kyowa Interface Science Co., Ltd. As one example, the pure water contact angle on the fluorine resin coating of the peripheral portion 112 is 115° to 118° whereas the pure water contact angle on the sensing film portion 111 of the PEN substrate is 70° to 80°.

### (4) Formation of Droplet Film from Polymer Adhesive Solution

First, the polymer adhesive solution prepared in the above (1) is dripped onto the sensing film portion 111 on which the patterning has previously been formed, and thereby a droplet film containing the polymer adhesive is formed. Specifically, 10 µL to 30 µL of the polymer adhesive solution (e.g., methyl alcohol in which HPC is dissolved) is dripped onto the sensing film portion 111 on which the patterning has been performed such that the diameter of the sensing film portion 111 is 8 mm.

When the dripping is performed, the first solvent of the polymer adhesive solution does not spread beyond the boundary between the liquid-repellent peripheral portion 112 and the lyophilic sensing film portion 111. Therefore, a polymer adhesive droplet film with little variation in its area can be realized. It is preferred to semi-dry the droplet film formed through the dripping. Even if the droplet film is fully dried, it does not affect the NO gas responsiveness. However, if the droplet film is fully dried, it increases a possibility of air bubbles being formed in the sensing film 11. In a case where air bubbles are formed in the sensing film 11, it is desired to perform vacuum defoaming by performing, for example, vacuum drawing on the sensing film at the time of dripping.

### (5) Formation of Droplet Film as Sensing Film

Next, 10 µL to 30 µL of the dye-containing preparation solution prepared in the above (2) is dripped onto the polymer adhesive droplet film. At the time, the preparation solution does not spread beyond the boundary between the sensing film portion 111 and the peripheral portion 112, and convection between the first solvent and the preparation solution occurs at the surface of the sensing film portion 111. As a result, a uniform droplet-based sensing film containing the nitric oxide sensing particles is formed. The reason for this is that the specific gravity of the alcohol-based solvent which is the first solvent is lighter than the halogen-based solvent which is the second solvent. In a case where the diameter of the sensing film portion 111 is 8 mm, if the dripping amounts of both the first solvent and the second solvent are 30 µL or less, then the droplet-based sensing film is not formed beyond the aforementioned boundary, and thus the sensing film 11 with little variation in its area is realized.

### (6) Drying and Fixing of Sensing Film

Next, the droplet-based sensing film on the substrate 12 is air-dried and solidified to form the sensing film 11. Temperature and humidity conditions for drying the droplet-based sensing film are not particularly limited. For example, the droplet-based sensing film may be dried by air drying (at a room temperature with a relative humidity of 50 %), or may be dried at a temperature higher than the room temperature, or may be dried by being heated up on a hot plate, so long as alteration of the substrate, the polymer adhesive, or the dye is not caused.

Preferably, the dripping amounts of the polymer adhesive solution and the dye-containing preparation solution are adjusted such that the weight ratio of the polymer adhesive to the inorganic particles is 0.07 g/g to 0.20 g/g. If the ratio of the polymer adhesive is lowered, the adhesion force between the nitric oxide sensing particles becomes weak, which tends to cause the particles to detach from the nitric oxide detection element. Although the adhesion force between the particles increases in accordance with an increase in the polymer adhesive ratio, such an increase in the polymer adhesive ratio tends to result in an extended NO response time.

Described next are specific examples of a NO detection method using the nitric oxide detection element 10A and detection results.

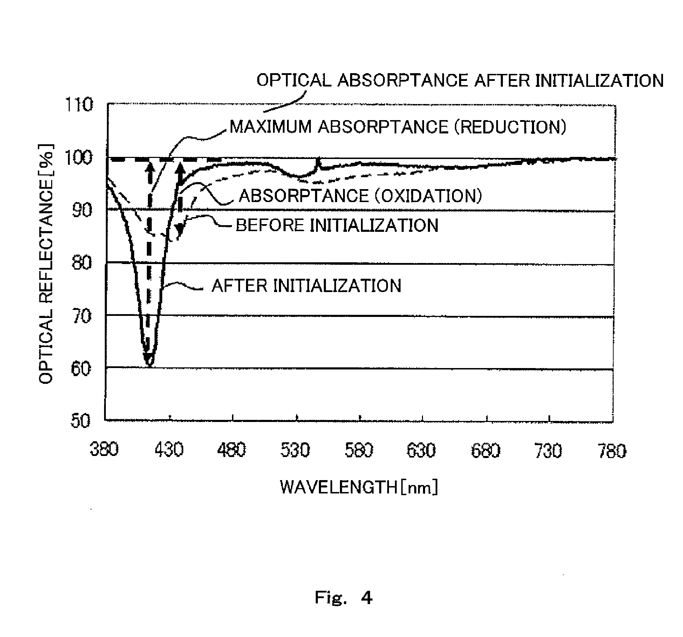

Fig. 4 is a graph showing a UV-Vis reflection spectrum which was obtained from a measurement using a spectrophotometer MCPD-7000 (available from Otsuka Electronics Co., Ltd.) and CoTP(4-OCH₃)P as a dye.

Prior to the measurement, heat treatment is performed for ten minutes to initialize the nitric oxide detection element 10A. In the initialization, nitrogen gas (flow rate 100 mL/min) is flowed to the nitric oxide detection element 10A, with a sensor temperature set to 150 °C by the temperature controller 24. Through such initialization by heat treatment, a reflection spectrum derived from CoTP(4-OCH₃)P of which the central metal is divalent cobalt (hereinafter, referred to as Co(II)TP(4-OCH₃)P) is obtained, and the reflection spectrum has an absorption band having a central wavelength of 413 nm (the spectrum is indicated as "AFTER INITIALIZATION" in Fig. 4).

Next, the measurement cell 13 is stabilized for ten minutes, during which nitrogen gas is flowed into the measurement cell 13 at a flow rate of 100 mL/min, with the sensor temperature set to 80 °C by the temperature controller 24. Thereafter, NO gas exposure is performed. When NO is bound to Co(II)TP(4-OCH₃)P, electrons in the d orbital of cobalt are coordinated while charge transfer to the unpaired electron orbital of NO is caused. As a result, the divalent cobalt is oxidized into trivalent cobalt. Consequently, the absorption band derived from Co(II)TP(4-OCH₃)P, which has a central wavelength of 413 nm, is attenuated as shown in Fig. 4. Also, an absorption band that is derived from CoTP(4-OCH₃)P containing trivalent cobalt (hereinafter, referred to as Co(III)TP(4-OCH₃)P) and that has a central wavelength of 438 nm (i.e., an absorption band in the spectrum indicated as "BEFORE INITIALIZATION" in Fig. 4) is increased. It should be noted that the present invention is not limited to the above specific sensor temperatures and treatment periods.

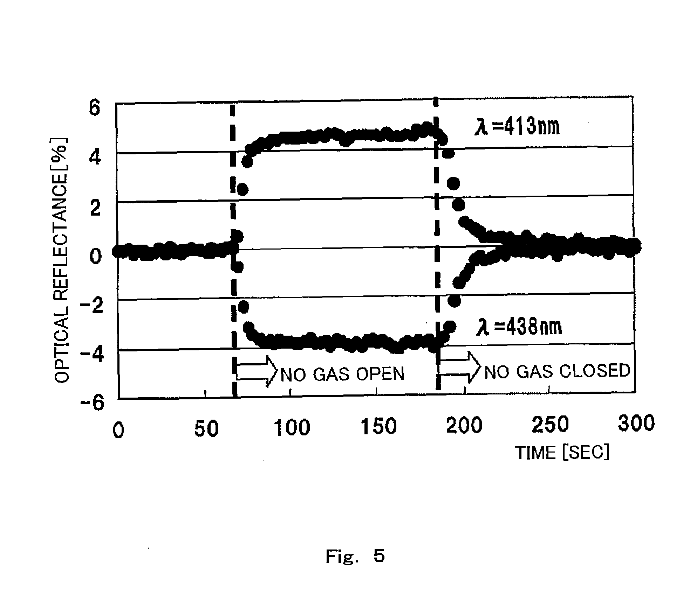

Fig. 5 is a graph showing changes in optical reflectance between before and after the initialized nitric oxide detection element according to the present invention is exposed to NO gas. Specifically, the graph shows changes in optical reflectance at the wavelength of 413 nm derived from Co(II)TP(4-OCH₃)P and in optical reflectance at the wavelength of 438 nm derived from Co(III)TP(4-OCH₃)P). An optical reflectance defined as 100 % in Fig. 5 is an optical reflectance at a wavelength of 470 nm in the light reflection spectrum of Fig. 4, at which wavelength the dye does not react with NO gas. Fig. 5 shows a value obtained by subtracting the optical reflectance at the optical wavelength 413 nm after the NO gas exposure from the optical reflectance at the optical wavelength 413 nm before the NO gas exposure, and a value obtained by subtracting the optical reflectance at the optical wavelength 438 nm after the NO gas exposure from the optical reflectance at the optical wavelength 438 nm before the NO gas exposure.

Conditions for the NO gas exposure at the time of the measurement are as follows: the sensor temperature is 80 °C; the concentration of nitrogen-diluted NO gas is 1 ppm-NO; and the flow rate of the NO gas is 200 ml/min. Hereinaftter, a value obtained by subtracting the saturation value of the optical reflectance at the wavelength 438 nm from the saturation value of the optical reflectance at the wavelength 413 nm at the time of NO gas exposure, is referred to as a "differential optical reflectance". The differential optical reflectance depends on the NO concentration. Therefore, the NO concentration can be determined based on the differential optical reflectance of CoTP(4-OCH₃)P, which occurs from the NO exposure.

The present invention requires the sensing film 11 to contain CoTP(4-OCH₃)P containing divalent cobalt reactive with NO gas. The sensing film 11 may also contain CoTP(4-OCH₃)P containing trivalent cobalt in addition to CoTP(4-OCH₃)P containing divalent cobalt. A reflection spectrum obtained in a case where CoTP(4-OCH₃)P contains both divalent cobalt and trivalent cobalt is such that the absorption band derived from Co(II)(4-OCH₃)P and having a central wavelength of 413 nm and the absorption band derived from Co(III)(4-OCH₃)P and having a central wavelength of 438 nm, as shown in the reflection spectrum of Fig. 4, are combined.

When the nitric oxide detection element 10A fabricated in the above-described method reacts with oxygen (O₂) and carbon monoxide (CO) in the atmosphere, Co(III)(4-OCH₃)P becomes a major cobalt component. This hinders precise NO concentration measurement. Therefore, it is desired that the initialization through the heat treatment is performed on the nitric oxide detection element 10A prior to the measurement.

Specifically, in order to realize precise measurement of the concentration of a trace amount of NO gas, the sensing film 11 is initialized immediately before the NO gas measurement. In the initialization, cobalt in CoTP(4-OCH₃)P is transformed into divalent cobalt. The sensing film 11 may be initialized through the above-described heat treatment, or may be initialized through light irradiation onto the sensing film 11 or electromagnetic irradiation onto the sensing film 11 by microwaves. Moreover, these methods may be combined to perform the initialization.

When CoTP(4-OCH₃)P is heated up, gases such as O₂ and CO bound to CoTP(4-OCH₃)P are desorbed, and cobalt in CoTP(4-OCH₃)P is reduced to divalent cobalt. At the time of heating up CoTP(4-OCH₃)P, air or an inert gas such as N₂ gas or Ar gas may be flowed. By flowing such a gas, the desorbed gases such as O₂ and CO can be efficiently removed from the inside of the measurement cell 13.

The temperature and period of heating by means of the temperature controller 24 may be set appropriately so that the dye, the polymer adhesive, and the substrate will not be degraded and so that the heat treatment can be performed quickly. In particular, the sensor temperature at the time of performing the heat treatment is preferably in a range from 50 °C to 200 °C. If the heating temperature in the initialization is less than 50 °C, then the heat treatment period is extended in accordance with a decrease in the heating temperature. If the sensor temperature exceeds 200 °C, it causes alteration of the polymer adhesive.

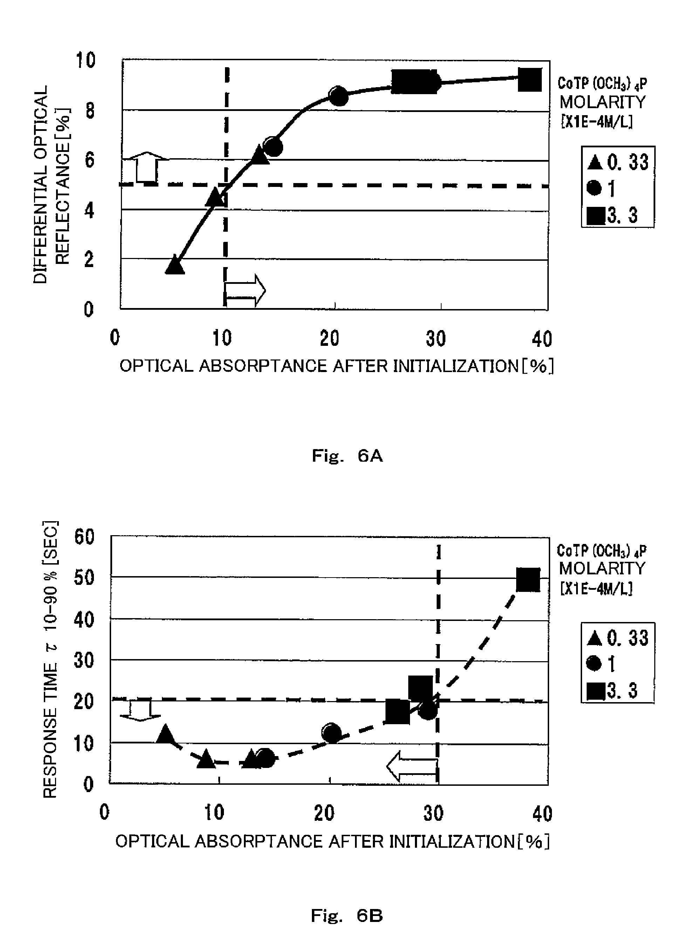

Fig. 6A and Fig. 6B are graphs showing a relationship between an optical absorptance after the heat treatment initialization and the NO sensitivity, and a relationship between the optical absorptance after the heat treatment initialization and the NO response time. The NO sensitivity is linear with respect to the differential optical reflectance. The differential optical reflectance that is represented by the vertical axis of Fig. 6A is a value obtained by subtracting "the saturation value of the reflectance at the wavelength of 438 nm" from "the saturation value of the reflectance at the wavelength of 413 nm" at the time of NO exposure shown in Fig. 5. An optical absorptance after initialization, represented by the horizontal axis, is obtained from the optical absorptance after the heat treatment initialization (= 100 % - minimum reflectance value). The optical absorptance reflects a loading amount of CoTP(4-OCH₃)P physically adsorbed onto the inorganic particles. That is, the less the optical absorptance, the less the loading amount, and the greater the optical absorptance, the greater the loading amount.

The vertical axis of Fig. 6B represents the NO response time, which is a period (seconds) required for 10 % to 90 % of the change in the differential optical reflectance at the wavelength of 413 nm shown in Fig. 5 to occur.

It is clear from Fig. 6A that, in a region where the optical absorptance after initialization is less than 20 %, there is a linear relationship between the optical absorptance and the NO sensitivity. In other regions where the optical absorptance after initialization exceeds 20 %, the NO sensitivity indicates a saturation trend.

According to asthma guidelines (American Thoracic Society Documents "ATS/ERS Recommendations for Standardized Procedures for the Online and Offline Measurement of Exhaled Lower Respiratory Nitric oxide and Nasal Nitric oxide, 2005"), it is required to detect and measure 2 ppb of NO gas in exhaled air within ten seconds. In view of this, performance requirements for a nitric oxide detection element to be excellent in determining asthma are as follows: the differential optical reflectance, which corresponds to the NO sensitivity, is 5 % or higher (first threshold); and the NO response time (10 % to 90 % value) is 20 seconds or shorter (second threshold). These requirements are applied as setting values in the description hereinafter. If the first threshold is satisfied, NO sensitivity requirements specified in the asthma guidelines can be met through a publicly known method as follows, that is: use an optical band-pass filter; increase the number of times of measurement sampling by an electrical circuit of the photodetector; and improve the signal/noise ratio of a detection circuit. In relation to the second threshold, the asthma guidelines specify a condition that the flow rate of exhaled air is 3000 mL/min. In the measurement conditions according to the present embodiment, the flow rate is 200 mL/min. Accordingly, with the flow rate specified by the asthma guidelines, collision probability between NO gas and divalent cobalt is increased by 15 times. If the NO response time of 20 seconds in the present embodiment is converted according to the condition specified by the guidelines, the NO response time becomes approximately 1.3 seconds. The above setting values are merely performance requirements for a nitric oxide detection element to be excellent in determining asthma. Even if a nitric oxide detection element fails to satisfy these setting values, it does not mean that the nitric oxide detection element is unusable.

It is clear from Fig. 6A that the optical absorptance that satisfies the first threshold, i.e., the optical absorptance that allows the differential optical reflectance corresponding to the NO sensitivity to be 5 % or higher, is 10 % or higher. It is clear from Fig. 6B that the optical absorptance that satisfies the second threshold, i.e., the optical absorptance that allows the NO response time to be 20 seconds or shorter, is 30 % or lower. If the optical absorptance exceeds 30 %, the NO response time becomes longer than 20 seconds. This is considered to be caused by dye aggregation. In view of these, in the present invention, the amount of CoTP(4-OCH₃)P loading on the inorganic particles is preferably such that the optical absorptance after initialization at an optical wavelength of 400 nm to 450 nm (Soret band) (maximum optical absorptance) is 10 % to 30 %.

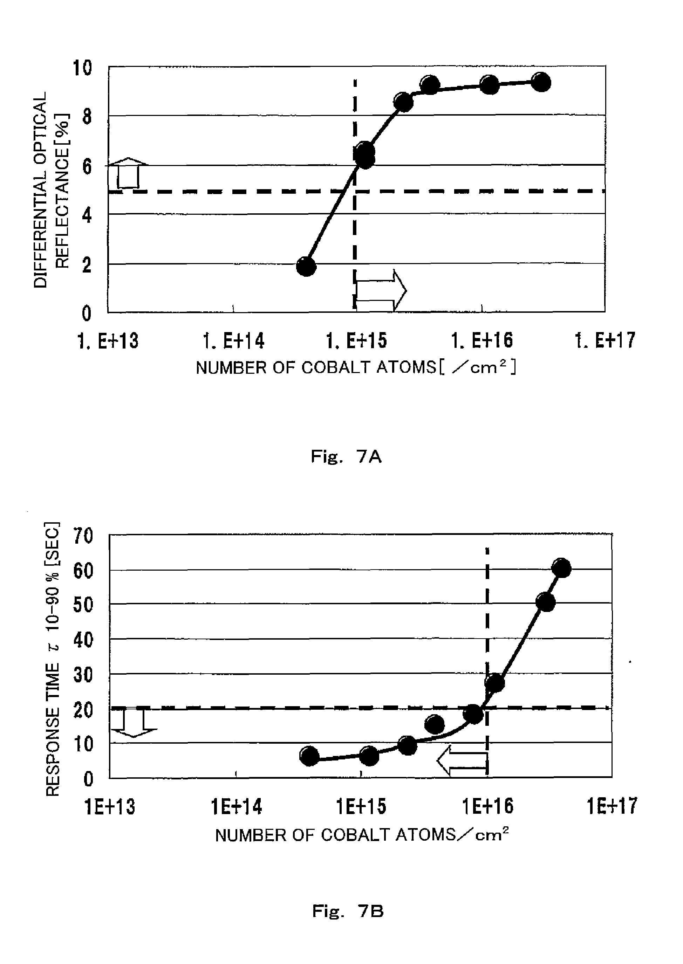

Fig. 7A and Fig. 7B show a relationship between the NO sensitivity and the number of cobalt atoms per unit area of the substrate surface of the nitric oxide detection element according to the present invention, and a relationship between the NO response time and the number of cobalt atoms per unit area of the substrate surface of the nitric oxide detection element according to the present invention. The number of cobalt atoms on the substrate surface can be measured by using a publicly known secondary ion mass spectrometer (abbreviated as SIMS). In Fig. 7A and Fig. 7B, the horizontal axis represents the number of cobalt atoms per unit area of the substrate surface, the surface area of which is defined by the above-described patterning and the property of which is varied by changing the CoTP(4-OCH₃)P concentration in the preparation solution. It is clear from Fig. 7A that the number of cobalt atoms is required to be 1×10¹⁵/cm² or more in order to satisfy the first threshold of the NO sensitivity. It is clear from Fig. 7B that the number of cobalt atoms is required to be 1×10¹⁶/cm² or less in order to satisfy the second threshold of the NO response time. In view of these, the number of cobalt atoms per unit area of the substrate surface is preferably 1×10¹⁵/cm² to 1×10¹⁶/cm². The NO sensitivity tends to decrease in accordance with a decrease in the number of cobalt atoms and become insufficient. The NO response time tends to be extended in accordance with an increase in the number of cobalt atoms. This is considered to be caused by dye aggregation.

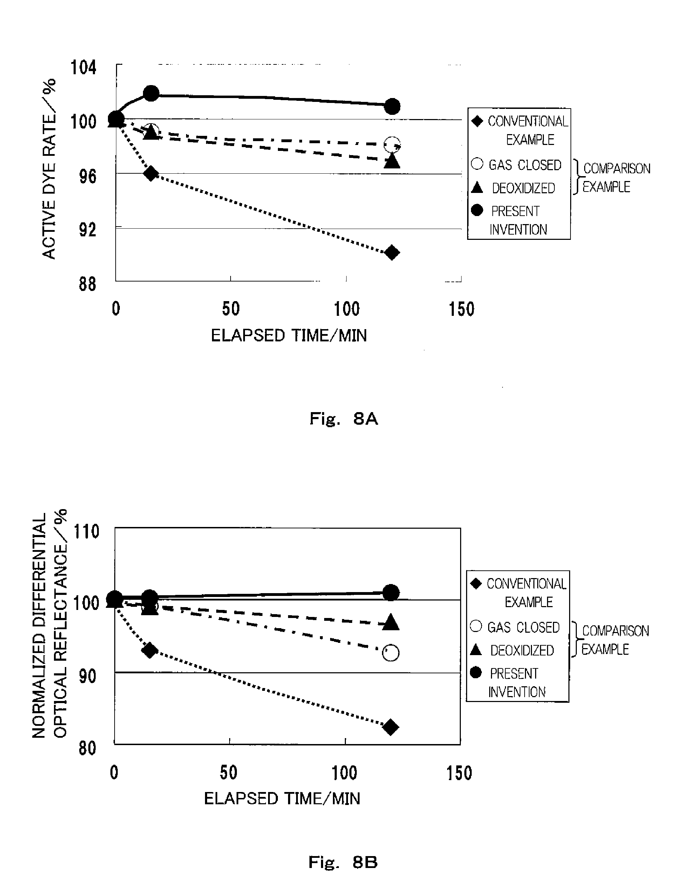

Fig. 8A and Fig. 8B are graphs showing temporal changes in an active dye rate and in a normalized differential optical reflectance which indicates a NO sensitivity. Both of the temporal changes were obtained from the optical absorptance after heat treatment of the nitric oxide detection element of the present invention. The following conditions were applied as accelerated conditions for dye degradation: the sensor temperature was set to 80 °C; the flow rate of air was set to 100 mL/min; and light having an optical wavelength range including 400 nm to 450 nm was emitted to irradiate the surface of the sensing film portion of the detection element with an intensity of 1 µW/cm² per unit area (in terms of a wavelength of 430 nm). While the surface was irradiated with the light, temporal changes in the optical absorptance and the differential optical reflectance of the nitric oxide detection element were evaluated.

The sensing film used in this measurement was fabricated with the following conditions: the molarity of CoTP(4-OCH₃)P in the preparation solution was 1×10⁻⁴ mol/L; the concentration of the non-ionic surfactant Triton X-100 in the preparation solution was 7.5 mg/mL; the concentration of water-repellent treated silica particles in the preparation solution was 30 mg/mL; and [molarity of the radical scavenger]/[molarity of CoTP(4-OCH₃)P] = 10 (molar ratio).

Figs. 8A and 8B show examples including: "CONVENTIONAL EXAMPLE" where a nitric oxide detection element was used, which was fabricated in the same manner as described above except for absence of the use of the radical scavenger; "GAS CLOSED" as a comparison example where the nitric oxide detection element according to the present invention was used with such operation conditions that only the above-described light irradiation was performed without flowing oxygen gas; and "DEOXYDIZED" as another comparison example where after NO gas exposure, a three-way valve (not shown) was set at the gas introducing inlet 14 of the measurement cell 13 and a tank (not shown) containing a deoxidizer Ageless (available from Mitsubishi Gas Chemical Company, Inc.) was connected thereto, and only the light irradiation was performed at an oxygen concentration of approximately 0.1 % (V/V).

In the respective cases shown in Figs. 8A and 8B, heat treatment was performed as initialization for ten minutes with air flowed at a flow rate of 100 mL/min and the sensor temperature set to 150 °C. After the initialization was performed, the sensor temperature was reduced to 80 °C for ten-minute stabilization and then the "optical absorptance" was measured. Initial values of the optical absorptance obtained in the respective cases at an elapsed time of 0 minute were normalized as 100 %. Temporal changes in each of the normalized optical absorptances are indicated in the graph as an "active dye rate". Also, in the above respective cases, the "differential optical reflectance" was obtained from reflectance changes occurring when the nitric oxide detection element was exposed to 1 ppm of NO gas (diluted gas: air) at a rate of 200 mL/min with the sensor temperature set to 80 °C, and initial values of the differential optical reflectance obtained in the respective cases were normalized as 100 %. Temporal changes in each of the normalized differential optical reflectances are indicated in the graph.

It is clear from Figs. 8A and 8B that when a radical scavenger is added to the sensing film, temporal changes in the optical absorptance (= active dye rate) and in the NO sensitivity (= normalized differential optical reflectance) are small, and threfore, time degradation in the performance of the nitric oxide detection element is suppressed.

It should be noted that it was found that among the samples in "PRESENT INVENTION", "CONVENTIONAL EXAMPLE", "GAS CLOSED", and DEOXYDIZED", there was a common tendency for the NO response time to be slightly reduced as time elapsed.

Next, Table 1 and Table 2 show temporal changes in the optical absorptance after the heat treatment initialization and temporal changes in the differential optical reflectance which is the NO sensitivity at the time of NO gas exposure. Table 1 and Table 2 show these temporal changes in multiples cases among which a [radical scavenger molarity]/[CoTP(4-OCH₃)P molarity] ratio was varied.

**[Table 1]**

| Optical Absorptance after Heating Treatment [%] | | | | |
|---|---|---|---|---|
| Sample | Radical Scavenger/Dye [Molar Ratio] | 0 Hour Elapsed | After 2 Hours | After 24 Hours |
| Conventional Example 1 | 0 | 21.3 | 16.6 | 6.0 |
| Experiment Example 1 | 0.1 | 23.8 | 21.5 | 8.3 |
| Experiment Example 2 | 0.3 | 23.5 | 21.8 | 11.4 |
| Experiment Example 3 | 1.0 | 24.8 | 24.1 | 23.6 |
| Experiment Example 4 | 10 | 22.7 | 22.2 | 22.1 |
| Experiment Example 5 | 100 | 20.4 | 20.6 | 20.2 |
| Experiment Example 6 | 300 | 20.1 | 20.2 | 20.0 |

**[Table 2]**

| NO Responsiveness | | Differential Optical Reflectance [%] | | |
|---|---|---|---|---|
| Sample | Radical Scavenger/Dye [Molar Ratio] | 0 Hours Elapsed | After 2 Hours | After 24 Hours |
| Conventional Example 1 | 0 | 8.5 | 7.0 | 0.8 |
| Experiment Example 1 | 0.1 | 10.1 | 9.0 | 1.8 |
| Experiment Example 2 | 0.3 | 9.9 | 8.7 | 5.4 |
| Experiment Example 3 | 1.0 | 9.7 | 9.5 | 9.2 |
| Experiment Example 4 | 10 | 9.6 | 9.5 | 9.4 |
| Experiment Example 5 | 100 | 10.5 | 10.3 | 10.0 |
| Experiment Example 6 | 300 | 9.8 | 9.3 | 9.3 |

Nitric oxide detection elements used in the above respective measurements were fabricated under the conditions as follows. Preparation solutions were prepared such that in each preparation solution, the molarity of CoTP(4-OCH₃)P was 1×10⁻⁴ mol/L chloroform, the concentration of the non-ionic surfactant Triton X-100 was 7.5 mg/mL chloroform, and the concentration of water-repellent treated silica particles having a particle diameter of 6 µm to 12 µm was 30 mg/mL chloroform. A radical scavenger PBN was added to the preparation solutions at respective ratios shown in Tables 1 and 2. A polymer adhesive solution was prepared such that the HPC concentration was 6 mg/mL methyl alcohol. First, patterning with a fluorine resin film was performed on the surface of the substrate 12 which is a plastic substrate PEN (having a thickness of 0.1 mm). The sensing film portion 111 was formed into a round shape having a diameter of 8mm, and a fluorine resin film was formed on the peripheral portion 112.

The polymer adhesive solution in an amount of 10 µL was dripped onto the sensing film portion 111 and then semi-dried for 30 seconds. In this manner, a polymer adhesive droplet film was formed. The preparation solution in an amount of 20 µL was dripped onto the polymer adhesive droplet film and then air-dried for three minutes. A droplet-based sensing film thus formed was further dried for 30 seconds at 50 °C on a hotplate. Accordingly, a sensing film was formed. In this manner, the nitric oxide detection elements were fabricated for the respective measurements. Nitric oxide detection element fabrication conditions were the same among these nitric oxide detection elements except for the amount of the radical scavenger to be added.

For the purpose of examining temporal changes in the optical absorptance and in the differential optical reflectance, settings were made as follows for an accelerated test for a radical production amount: the sensor temperature was set to 80 °C; the oxygen gas flow rate was set to 100 mL/min; and light having a wavelength range including 400 nm to 450 nm was used as irradiation light, the intensity of which was set to 1 µW/cm² in terms of an optical wavelength of 430 nm. Irradiation with the light was performed and NO gas exposure experiments were performed 0 hour (initial value), 2 hours, and 24 hours after the light irradiation. In the NO gas exposure experiments, the "optical absorptance" was measured after ten-minute heat treatment initialization and ten-minute stabilization, the ten-minute heat treatment initialization being performed with a sensor temperature of 150 °C and an air flow rate of 100 mL/min and the ten-minute stabilization being performed with a sensor temperature of 80 °C and an air flow rate of 100 mL/min, and then, the "differential optical reflectance" was measured when exposure to 1 ppm of air-diluted NO gas (at a flow rate of 200 mL/min) was performed with a sensor temperature of 80 °C.

It is clear from Table 1 and Table 2, time degradation in the optical absorptance and the differential optical reflectance was suppressed owing to the addition of the radical scavenger.

The value of [radical scavenger]/[dye] (molar ratio) is preferably 0.3 to 100. As shown in Experiment Example 6, if the value of [radical scavenger]/[dye] is in a range exceeding 100, it causes a reduction in NO response speed although time degradation in the optical absorptance and the NO sensitivity is suppressed. The reason for this is considered that NO gas reaches the dye after spreading within the radical scavenger since the dye is supported in a dispersed manner on the radical scavenger added by a large amount. Moreover, as shown in Experiment Example 1, if the value of [radical scavenger]/[dye] is in a range less than 0.3, time degradation in the optical absorptance and the NO sensitivity is suppressed as compared to Conventional Example 1 where no radical scavenger is added. In this case, however, after 24 hours, the optical absorptance requirement of 10 % to 30 % and the differential optical reflectance requirement of 5 % or higher, which are set in accordance with the asthma guidelines, are not met. Therefore, in order to obtain favorable NO responsiveness and meet the optical absorptance and differential optical reflectance requirements set in accordance with the asthma guidelines, the value of [radical scavenger]/[dye] (molar ratio) is preferably 0.3 to 100.

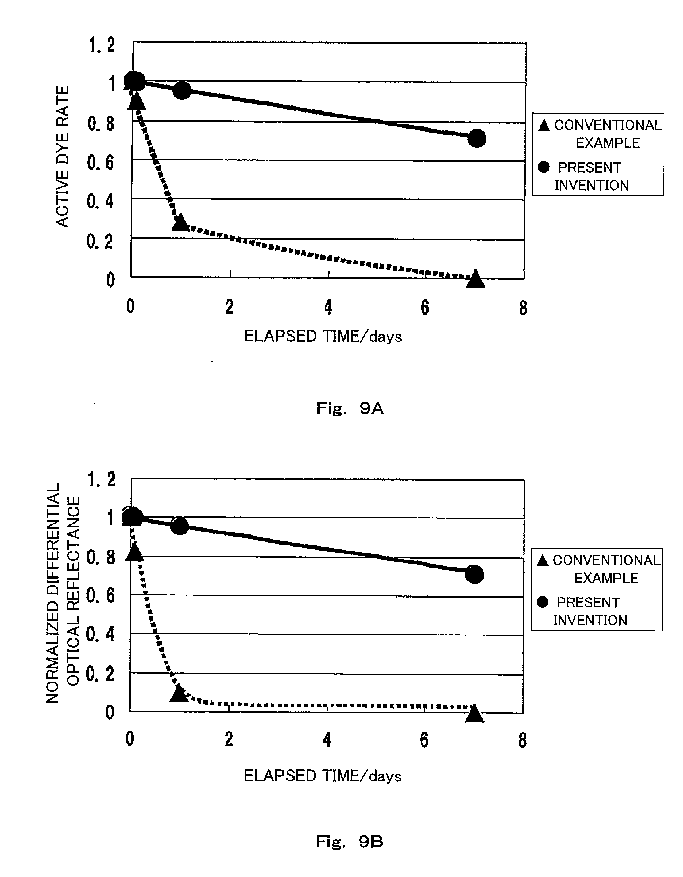

Fig. 9A and Fig. 9B are graphs showing temporal changes in the active dye rate and the normalized differential optical reflectance of the nitric oxide detection element.

Here, the nitric oxide detection element was fabricated under the same conditions as those in the case of Table 1 and Table 2 except that the value of [radical scavenger]/[dye] (molar ratio) was set to 10. Measurement was performed by using the nitric oxide detection element. In the measurement, temporal changes in the active dye rate and the normalized differential optical reflectance of the nitric oxide detection element were checked at elapsed times of 0 day, 1 day, and 7 days.

It is clear from Fig. 9A and Fig. 9B that adding a radical scavenger to the sensing film of the nitric oxide detection element is significantly effective for suppressing time degradation. Even after seven days elapsed, the performance of the nitric oxide detection element is approximately 70 % of the initial values.

It should be noted that if a deoxidizer is used instead of a radical scavenger, then the amount of accumulated oxygen gas is restricted due to the oxygen absorbing capacity of the deoxidizer. For this reason, the use of a deoxidizer is not suitable in the case of suppressing time degradation while flowing oxygen gas for a long period of time.

### (Embodiment 2)

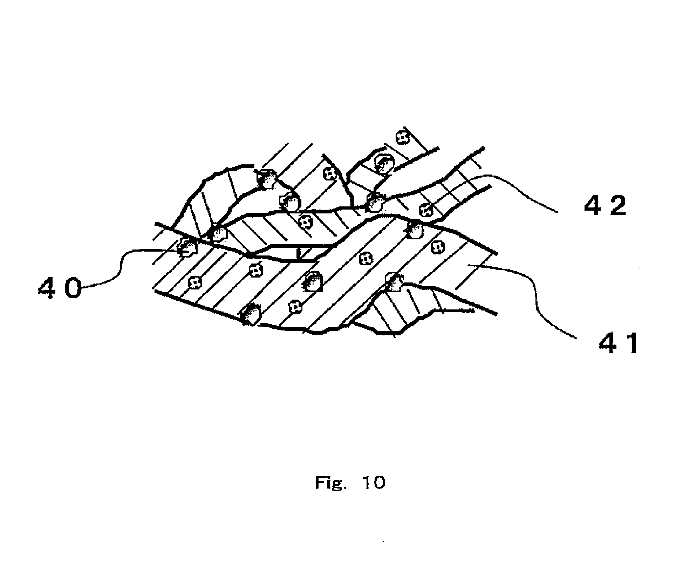

Fig. 10 is an enlarged conceptual diagram showing a relationship among materials forming a nitric oxide detection element according to Embodiment 2. In Embodiment 2, a dye 40 and a radical scavenger 42 are directly supported on the surface of a support 41. The dye and the radical scavenger are the same as those described in Embodiment 1. Similar to Embodiment 1, a non-ionic surfactant may be used in Embodiment 2. However, the use of a non-ionic surfactant is not essential.

For example, filter paper, a nonwoven fabric, or a woven fabric may be used as the support 41. Examples of a material forming the filter paper include a cellulosic material, a non-cellulosic material, glass fiber, PTFE, and a mixture of these. Preferably, the nitric oxide detection element according to the present invention exhibits an optical absorptance of 10 % to 30 % in the Soret band; the NO sensitivity of the nitric oxide detection element is a differential optical reflectance of 5 % or higher; and the NO response time is 20 seconds or shorter. Therefore, the material of the filter paper may be selected so that these physical properties can be satisfied.

Hereinafter, an example of a method of fabricating the nitric oxide detection element according to Embodiment 2 is described.

CoTPP in which Xi = H was added as the dye 40 to a chloroform solvent and PBN was added as the radical scavenger 42 to the chloroform solvent. Then, a preparation solution was prepared such that the molarity of CoTPP was 3.3×10⁻⁵ mol/L to 3.3×10⁻⁴ mol/L and [PBN]/[CoTPP] = 0 to 300 (molar ratio). Filter paper 5A (available from ADVANTEC) was used as a substrate 41. The nitric oxide detection element was fabricated by fixing the filter paper 5A to a stainless steel frame (not shown) having a rectangular shape, and dripping 10 µL of the preparation solution onto the filter paper 5A by means of a micropipette.

In Embodiment 2, conditions for the heat treatment initialization, conditions for the NO gas exposure, and accelerated conditions for the radical production amount are the same as those in Embodiment 1.

Also in Embodiment 2, it has been confirmed that the use of both of a dye and a radical scavenger makes it possible to suppress time degradation in the performance of the nitric oxide detection element. In particular, it has been confirmed that in the case of satisfying [PBN]/[CoTPP] = 0.3 to 100 (molar ratio), the nitric oxide detection element satisfies the following requirements: the optical absorptance is 10 % to 30 % in the Soret band; and the NO sensitivity is a differential optical reflectance of 5 % or higher, and also, the nitric oxide detection element is capable of measuring the concentration of a trace amount of NO gas in a scale of ppb.

### (Embodiment 3)

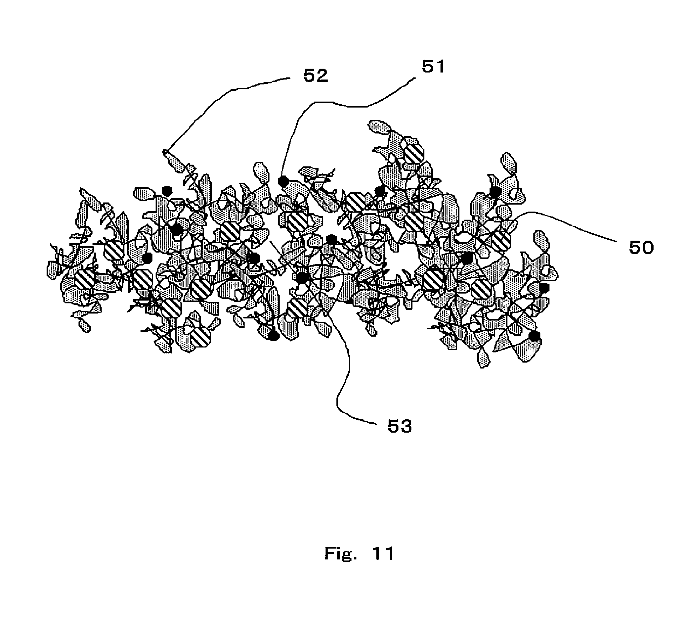

Fig. 11 is an enlarged conceptual diagram showing a relationship among materials forming a nitric oxide detection element according to Embodiment 3. In Embodiment 3, inorganic particles are not used, and a dye 50 and a radical scavenger 51 are dispersed within a polymer adhesive 52. A sensing film made of such a polymer adhesive 52 is formed on the surface of the substrate 12 (not shown). In Embodiment 3, the same dye, radical scavenger, polymer adhesive, and substrate as those described in Embodiment 1 are used.

Hereinafter, an example of a method of fabricating the nitric oxide detection element according to Embodiment 3 is described.

CoTP(4-OCH₃)P in which Xi = OCH₃ as the dye 50 was dissolved in a chloroform solvent; DMPO as the radical scavenger 51 was dissolved in the chloroform solvent; and PEO as the polymer adhesive 52 was dissolved in the chloroform solvent. In this manner, a preparation solution was prepared. At the time, the dye concentration was adjusted to 3.3×10⁻⁵ mol/L to 3.3×10⁻⁴ mol/L; [DMPO]/[CoTPP] was adjusted to 0 to 300 (molar ratio); and the concentration of the polymer adhesive PEO (having a molecular weight of 100 K) was adjusted to 0.1 % (g/V). An aluminum metal plate was used as the substrate 12. The sensing film 11 was formed on the substrate 12 by spin coating, such that the sensing film 11 had a thickness of 2 µm. In this manner, the nitric oxide detection element was fabricated.

In Embodiment 3, conditions for the heat treatment initialization, conditions for the NO gas exposure, and accelerated conditions for the radical production amount are the same as those in Embodiment 1.

In Embodiment 3 where the dye 50 and the radical scavenger 51 are supported in a dispersed manner within the polymer adhesive 52, rates of degradation in the optical absorptance and the differential optical reflectance are less than those in Embodiment 1 and Embodiment 2. It is presumed that this is because the dye 50 exists in the polymer adhesive 52 and in a gap 53 of the polymer adhesive 52, and also because, in Embodiment 3, the amount of the dye 50 that is exposed at the surface of the nitric oxide detection element (i.e., the surface of the gap 53) is less than in Embodiments 1 and 2.

Also in Embodiment 3, it has been confirmed that the use of both of a dye and a radical scavenger makes it possible to suppress time degradation in the performance of the nitric oxide detection element. In Embodiment 3, the NO response time is longer than in Embodiments 1 and 2. However, it has been confirmed that in Embodiment 3, particularly in the case of satisfying [DMPO]/[CoTPP] = 0.1 to 100 (molar ratio), the nitric oxide detection element satisfies the following requirements: the optical absorptance is 10 % to 30 % in the Soret band; and the NO sensitivity is a differential optical reflectance of 5 % or higher, and also, the nitric oxide detection element is capable of measuring the concentration of a trace amount of NO gas in a scale of ppb.

### (Embodiment 4)

In Embodiment 4, CoTP(4-OH)P in which Xi = OH is used as the dye 102; BHPBN is used as the radical scavenger 105; and Triton X-100 is used as the non-ionic surfactant 104. These were dissolved into methyl alcohol which was the second solvent, and thus a preparation solution was prepared. Here, the concentration of CoTP(4-OH)P was adjusted to 3.3×10⁻⁵ mol/L to 3.3×10⁻⁴ mol/L, and [BHPBN]/[CoTP(4-OH)P] was adjusted to 0 to 300 (molar ratio). The other fabrication conditions were the same as those in Embodiment 1.

In Embodiment 4, conditions for the heat treatment initialization, conditions for the NO gas exposure, and accelerated conditions for the radical production amount are the same as those in Embodiment 1.

Also in Embodiment 4, it has been confirmed that the use of both of a dye and a radical scavenger makes it possible to suppress time degradation in the performance of the nitric oxide detection element. In particular, in the case of satisfying [BHPBN]/[CoTP(OH)P] = 0.3 to 100 (molar ratio), the nitric oxide detection element satisfies the following requirements: the optical absorptance is 10 % to 30 % in the Soret band; and the NO sensitivity is a differential optical reflectance of 5 % or higher, and also, the nitric oxide detection element is capable of measuring the concentration of a trace amount of NO gas in a scale of ppb.

As described above, a nitric oxide detection element that is capable of measuring a trace amount of NO gas with high sensitivity and of which the time degradation in performance is suppressed can be realized by disposing, on the surface of the nitric oxide detection element, a radical scavenger and a dye containing a porphyrin of which the central metal is divalent cobalt. Since the present invention suppresses performance degradation caused by radical production due to oxygen, the present invention can be effectively applied to a nitric oxide detection element that detects NO gas in a mixed gas containing oxygen.

### Industrial Applicability

The nitric oxide detection element according to the present invention is useful for nitric oxide gas detection in the medical and pharmaceutical fields, drug development, environmental measurement, and chemical safety assessment.

### Reference Signs List

| | |
|---|---|
| 10A | nitric oxide detection element |
| 11 | sensing film |
| 12 | substrate |
| 13 | measurement cell |
| 14 | gas inlet |
| 15 | gas outlet |
| 16 | light source |
| 17 | photodetector |
| 18 | phototransmitter/receiver |
| 19 | measurement controller |
| 20, 21 | optical fiber |
| 22, 23, 25 | control line |
| 24 | temperature controller |
| 30 | measurement gas |
| 40, 50, 102 | dye |
| 41 | support |
| 42, 51, 105 | radical scavenger |
| 52 | polymer adhesive |
| 53 | free volume |
| 100 | nitric oxide sensing particle |
| 101 | inorganic particle |
| 103 | polymer adhesive |
| 104 | non-ionic surfactant |
| 111 | sensing film portion |
| 112 | peripheral portion |

## Claims

1. A nitric oxide detection element comprising at a surface thereof:
a dye having a porphyrin skeleton and containing divalent cobalt as a central metal; and
a radical scavenger.

2. The nitric oxide detection element according to claim 1, wherein the radical scavenger is a nitron compound.

3. The nitric oxide detection element according to claim 1, wherein a molar ratio of the radical scavenger to the dye is 0.3 to 100.

4. The nitric oxide detection element according to claim 1, comprising a substrate and a sensing film formed on a surface of the substrate, wherein
the dye and the radical scavenger are contained in the sensing film.

5. The nitric oxide detection element according to claim 4, wherein the substrate is a plastic substrate, a ceramic substrate, a metal substrate, paper, a woven fabric, or a nonwoven fabric.

6. The nitric oxide detection element according to claim 4, wherein
the sensing film is formed from nitric oxide sensing particles and a polymer adhesive, and
the nitric oxide sensing particles are formed in such a manner that the dye and the radical scavenger are adsorbed to surfaces of inorganic particles.

7. The nitric oxide detection element according to claim 6, wherein the inorganic particles are silica particles, α-alumina particles, or a mixture of silica particles and α-alumina particles.

8. The nitric oxide detection element according to claim 6, wherein the polymer adhesive has a glass transition temperature which is not lower than -150 °C and not higher than 150 °C.

9. The nitric oxide detection element according to claim 4, wherein the sensing film is formed in such a manner that the dye and the radical scavenger are dispersed within a polymer adhesive.

10. The nitric oxide detection element according to claim 1, further comprising a support, wherein
the dye and the radical scavenger are supported on a surface of the support.

11. The nitric oxide detection element according to claim 10, wherein the support is filter paper, a nonwoven fabric, or a woven fabric.

12. The nitric oxide detection element according to claim 1, wherein
the dye is cobalt tetraphenylporphyrin, cobalt tetramethoxyphenylporphyrin, or a mixture of these compounds.

13. The nitric oxide detection element according to claim 1, wherein the dye is cobalt tetrahydroxyphenylporphyrin.

14. The nitric oxide detection element according to claim 1, wherein a maximum optical absorptance of the nitric oxide detection element in the Soret band is not lower than 10 % and not higher than 30 %.

15. The nitric oxide detection element according to claim 1, wherein the number of cobalt atoms per unit area of the surface which contains the dye is not less than 10¹⁵/cm² and not more than 10¹⁶/cm².

16. The nitric oxide detection element according to claim 1, wherein at a time of performing nitric oxide detection, the surface which contains the dye is irradiated with light having an optical wavelength not shorter than 400 nm and not longer than 450 nm.

17. A nitric oxide detector comprising:
the nitric oxide detection element according to claim 1;
a gas introducing part configured to cause a measurement gas, which possibly contains nitric oxide, to come into contact with the surface of the nitric oxide detection element;
a phototransmitter configured to emit light to the surface; and
a photoreceiver configured to receive light reflected by the surface or light transmitted through the surface.

18. A nitric oxide detection method comprising:
a first step of initializing the nitric oxide detection element according to claim 1;
a second step of emitting light to the surface of the nitric oxide detection element and measuring an optical absorptance of the surface after the first step;
a third step of causing a measurement gas, which possibly contains nitric oxide, to come into contact with the surface of the nitric oxide detection element after the second step;
a fourth step of emitting light to the surface and measuring an optical absorptance of the surface after the third step; and
a fifth step of comparing the optical absorptance obtained in the fourth step and the optical absorptance obtained in the second step to determine a nitric oxide concentration in the measurement gas.
